# EUROPEAN PATENT SPECIFICATION

(11) **EP 0 712 860 B1**
(45) Date of publication and mention of the grant of the patent: **05.12.2001**
(21) Application number: 95308034.8
(22) Date of filing: 09.11.1995
(51) Int. Cl.: C07H 19/073, C07H 19/06

(54) **Process for purifying and isolating 2'-deoxy-2',2'-difluoronucleosides**
Verfahren zur Isolierung und Reinigung von 2'-Deoxy-2',2'-difluornucleosiden
Procédé pour l'isolement et la purification de 2'-déoxy-2',2'-difluoro-nucléosides

(30) Priority: 17.11.1994 US 340972
(43) Date of publication of application: 22.05.1996
(73) Proprietor: ELI LILLY AND COMPANY, Indianapolis, Indiana 46285 (US)
(72) Inventor: Chou, Ta-Sen, Indianapolis, Indiana 46236 (US); Poteet, Laurie Michelle, Zionsville, Indiana 46077 (US); Kjell, Douglas Patton, West Lafayette, Indiana 47906 (US)
(74) Representative: Tapping, Kenneth George

(56) References cited:
- EP-A- 0 577 303
- US-A- 3 721 664
- US-A- 4 182 859
- US-A- 4 211 773
- US-A- 5 223 608

## Description

The invention pertains to the field of pharmaceutical chemistry and provides a process for purifying and isolating 2'-deoxy-2',2'-difluoronucleosides.

The continued interest in the synthesis of 2'-deoxynucleosides and their analogues is reflected in their successful use as therapeutic agents in viral and cancerous diseases. A critical step in the overall synthesis of 2'-deoxynucleosides is purification and isolation of the desired beta-anomer form of the nucleoside. This is critical because processes for synthesizing of 2'-deoxynucleosides are typically non-stereoselective and form a mixture of alpha and beta nucleosides.

Vorbruggen, et al., J. Org. Chem., 41, 2084 (1976), M. Hofer, Chem. Ber, 93, 2777 (1960), Walker, et al., Nucleic Acid Research, 12, 6827 (1984), R. P. Hodge et. al., J. Org. Chem., 56, 1553 (1991), Tann, et. al., J. Org. Chem., 50, 3644 (1985), Howell, et. al., J. Org. Chem., 53, 85 (1988) and U.S. Patent 4,965,374, Chou, et al., all report various syntheses of a mixture of alpha and beta anomer deoxy nucleosides.

EP-A-5 77 303 discloses a process to recover the beta-anomer of 2'-deoxy-2',2'-difluorocytidine wherein ethylacetate is used as a solvent.

Despite the processing advances in nucleoside synthesis, there continues to be a need for a process capable of efficiently purifying and isolating beta-anomer enriched 2'-deoxy-2',2'-difluoronucleosides in increased yields, wherein the difluoronucleosides have been synthesized in the absence of a catalyst.

Accordingly, the object of the present invention is to provide a process for efficiently purifying and isolating beta-anomer enriched 2'-deoxy-2',2'-difluoronucleosides.

Other objects and advantages of the present invention will become apparent from the following description of embodiments.

This invention is a process to purify and isolate a beta-anomer enriched nucleoside comprising:
a) providing a mixture containing R" and a beta-anomer enriched nucleoside of the formula 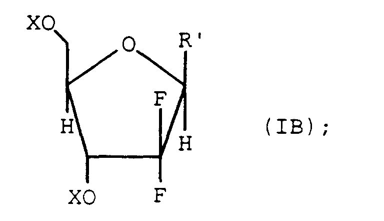 wherein each X is independently selected from hydroxy protecting groups and R' is a nucleobase of the formula 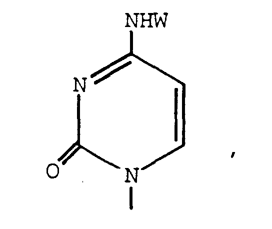 where W is an amino protecting group; and
   R" is a nucleobase of the formula 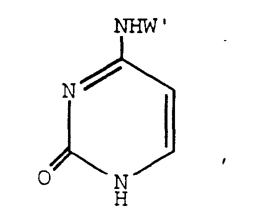 where W' is an amino protecting group or hydrogen; in a high boiling solvent;
b) diluting the mixture with acetonitrile;
c) adding the diluted reaction mixture to aqueous acid; and
d) holding the acid mixture so prepared at a temperature from 70°C to 100°C until the product of formula IB, where W is now W', has precipitated.

Throughout this document, all temperatures are in degrees Celsius, all proportions, percentages and the like are in weights and units and all mixtures are in volume units, except where otherwise indicated. Anomeric mixtures are expressed as a weight/weight ratio or as a percent. The term "alkyl" alone or in combination refers to straight, cyclic and branched chain aliphatic hydrocarbon groups which preferably contain up to 7 carbon atoms, such as methyl, ethyl, n-propyl, isopropyl, n-butyl, t-butyl, n-pentyl, n-hexyl, 3-methylpentyl groups and the like or substituted straight, cyclic and branched chain aliphatic hydrocarbons, such as chloroethyl, 1,2-dichloroethyl, and the like. The term "substituted" alone or in combination refers to a substitution by one or more of the groups selected from cyano, halo, carboalkoxy, toluoyl, nitro, alkoxy, alkyl, and dialkylamino. The phrase "anomer-enriched" alone or in combination refers to an anomeric mixture wherein the ratio of a specified anomer is greater than 1:1 and includes substantially pure anomer.

Hydroxy protecting groups (X) refer to hydroxy protecting groups known in the art, as described in Chapter 3 of Protective Groups in Organic Chemistry, McOmie Ed., Plenum Press, New York (1973), and Chapter 2 of Protective Groups in Organic Synthesis, Green, John, J. Wiley and Sons, New York (1981). Preferred hydroxy protecting groups are ester forming groups such as formyl, acetyl, substituted acetyl, propionyl, butynyl, pivaloyl, 2-chloroacetyl, benzoyl, substituted benzoyl, phenoxycarbonyl, methoxyacetyl; carbonate derivatives such as phenoxycarbonyl, ethoxycarbonyl, t-butoxycarbonyl, vinyloxycarbonyl, 2,2,2-trichloroethoxycarbonyl and benzyloxycarbonyl; alkyl ether forming groups such as benzyl, diphenylmethyl, triphenylmethyl, t-butyl, methoxymethyl, tetrahydropyranyl, allyl, tetrahydrothienyl, 2-methoxyethoxy methyl; carbamates such as N-phenylcarbamate and N-imidazoylcarbamate; more preferred are benzoyl, mono-substituted benzoyl and disubstituted benzoyl, acetyl, pivaloyl, triphenylmethyl ethers, and most preferred is benzoyl.

Amino protecting groups (W) are selected from the group consisting of silyl amine forming groups such as trialkylsilyl, including trimethylsilyl; isopropyldialkylsilyl, alkyldiisopropylsilyl, triisopropylsilyl, 1,1,3,3-tetraisopropyldisloxanyl, t-butyldialkylsilyl and t-butyldiarylsilyl; carbamates such as t-butoxycarbonyl, benzyloxycarbonyl, 4-methoxybenzyloxycarbonyl, and 4-nitrobenzyloxycarbonyl; formyl, acetyl, benzoyl and pivalamido; ether forming groups such as methoxymethyl, t-butyl, benzyl, allyl and tetrahydropyranyl; a preferred amino protecting group is trimethylsilyl.

The first step in the process of the claimed invention is to provide a mixture containing R" and a beta-anomer enriched nucleoside of the formula 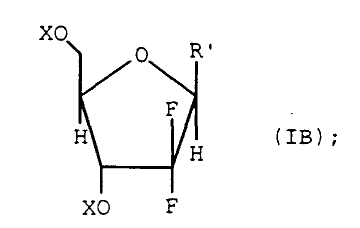 wherein each X is independently selected from hydroxy protecting groups and R' is a nucleobase of the formula 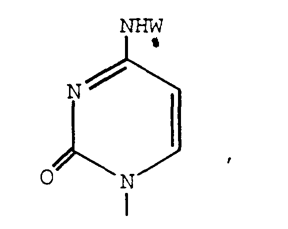 where W is an amino protecting group; and
R" is a nucleobase of the formula 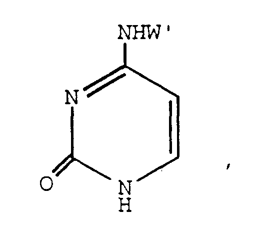 where W' is an amino protecting group or hydrogen; in a high boiling solvent.

Such a mixture may be created in many different ways. Methods to synthesize such a mixture are described and claimed in European Patent Application No. 577303 A1. By following certain of the processes (not using a catalyst; using an excess of R" nucleobase, pg. 9 EP-A-577303 ; and using a high-boiling solvent, page 10, lines 13-17) described in European Patent Application No. 577303 A1. it is possible to prepare the beta-anomer enriched nucleosides in an alpha to beta anomeric ratio of from greater than 1:1 to less than or equal to 1:9. A large excess of R" must be used to obtain these ratios. The excess R" is separated from the desired product by the process of the current invention.

The high boiling solvent is a solvent that has a boiling point above 70°C. The high boiling solvent is moderately polar, acid stable and non-nucleophilic. Typical high boiling solvents are aromatic haloalkyl, alkoxy and halo substituted,aromatic solvents, and mixtures thereof. Preferred high boiling solvents are 1,2-dichloroethane, 1,1,2-trichloroethane, glyme, diglyme, toluene, xylenes, anisole, dichlorobromomethane, chlorobenzene, dibromochloromethane, tribomomethane, dibromomethane, acetonitrile, propionitrile, dioxane and mixtures thereof, while more preferred is anisole.

Once a mixture containing R" and the described beta-anomer enriched nucleoside is provided, the process of the present invention proceeds as follows. First, the reaction mixture is diluted with the organic solvent acetonitrile. The dilution is carried out at an elevated temperature, which may be the reaction temperature. The organic solvent should be heated to an elevated temperature, as well, and the temperature of both the reaction mixture and the solvent should be in the range from 70°C to 110°C.

The amount of acetonitrile added is in the range from 1 ml to 5 ml per 1 gram of R" (protected or unprotected cytosine) used. No particular holding period is required after dilution of the reaction mixture; the diluted mixture may be taken immediately to the next step. The diluted reaction mixture is added to a large amount of aqueous acid at an elevated temperature. The purpose of the aqueous acid is to dissolve the excess R" (protected or unprotected cytosine), which was used in the glycosylation reaction described on page 9 of EP application No. 577 303 A1. Therefore, the amount and degree of acidity of the aqueous acid depends on the excess amount of R" (protected or unprotected cytosine) used in the reaction itself. Further, the amount of aqueous acid also depends on the choice of acid substance used in preparing the aqueous acid.

The most preferred acid is hydrochloric acid, used at a concentration from 1 N to 6 N, most preferably at 4 N. When that acid is used, and the amount of excess R" (protected or unprotected cytosine) is in the range from 5 X to 20 X, the amount of aqueous hydrochloric acid is from 3 ml to 5 ml per 1 gram of R" (protected or unprotected cytosine) used.

However, other aqueous mineral acids and conditions are also usable and may be preferred in various circumstances. For example, mineral acid substances such as sulfuric acid, sulfurous acid, phosphoric acid, nitric acid, and phosphonic acid may be used if desired by the operator. The concentration of the acid can be varied rather widely, approximately in inverse proportion to the acceptable volume of the total isolation step. In general, concentrations from 1 N to 10% can be used in the aqueous acid. The volume of aqueous acid must be optimized experimentally for the individual acid and the amount in the reaction mixture. The experiments needed are very simple, requiring the operator only to make successive adjustments of acid concentration and volume with the specific reaction mixture in use, observing the solubility of the R" (protected or unprotected cytosine) in each case.

The aqueous acid need not be hot when it is combined with the reaction mixture. The aqueous acid may be at ambient temperature, so long as the complete mixture is warmed to a temperature in the range from 70°C to 100°C. The heat of reaction may be sufficient to bring the mixture to that temperature, or it may be necessary in some cases to heat the mixture externally. In some cases, the heat of reaction is greater than 100°C, so the reaction mixture must be cooled so that it stays below 100°C. This is so because it is important that the temperature of the aqueous acid not be so elevated that any of the protecting groups are prematurely cleaved at this step of the process. When acetonitrile is used as the organic solvent, the most highly preferred temperature is from 70°C to 80°C.

The acidic mixture resulting from addition of the diluted reaction mixture to the aqueous acid is held, preferably with moderate agitation, for a period of time. The physical changes which occur during this holding period are the dissolution of the excess R" (protected or unprotected cytosine) in the aqueous acidic layer, and the precipitation of the desired beta-nucleoside. The precipitation is selective, and the undesired alphanucleoside remains in large part dissolved in the organic layer. Thus, the acidic mixture must be held, at constant temperature, until those two physical changes have occurred. In general, a period of from 10 minutes to 1 hour is adequate.

After a sufficient holding period, the precipitated beta-nucleoside is separated from the two liquid phases by filtration or centrifugation, and is washed with additional aqueous acid. The filtration or centrifugation should be carried out at approximately constant temperature to prevent dissolved R" (protected or unprotected cytosine) from precipitating out of solution. Beta-nucleoside isolated and purified in this manner may still have an amino protecting group (W) present, or the amino protecting group (W) may have been cleaved, and replaced with a hydrogen atom. Beta-nucleoside isolated and purified in the above manner is of superior purity, with respect to alpha-nucleoside, R" (protected or unprotected cytosine) and other impurities, and it is also found that the desired product is prepared in superior yield.

When the acidic mixture has been filtered or centrifuged to remove the solid product, the organic and aqueous layers of the filtrate are separated. The excess R" (protected or unprotected cytosine) is in the aqueous layer and may be removed from that layer and recycled back into the process. R" (protected or unprotected cytosine) may be recovered simply by cooling the aqueous layer and filtering the precipitated R" (protected or unprotected cytosine), or by making the aqueous layer basic, cooling the basic solution, and filtering to collect the precipitated R" (protected or unprotected cytosine). Recovered cytosine from the above process is routinely recycled into the process of making the mixture as described in EP No. 577 303 A1. Thus, the claimed process provides for economical recycling of R" (protected or unprotected cytosine), which is a beneficial property of the process.

The final phase of the process is the removal of the protecting groups X and any remaining W from the blocked purified, solid nucleoside of formula IB. The same anomeric ratio of unprotected nucleoside is obtained by removal of the protecting groups.

Most silyl-amino protecting groups are easily cleaved by use of a protic solvent, such as water or an alcohol. Most silyl-amino protecting groups are vulnerable to cleaving during contact with mineral acid. The acyl protecting groups, such as benzoyl and the acyl-amino protecting groups, are removed by hydrolysis with a strong base at a temperature from 0°C to 100°C. Strong or moderately strong bases suitable for use in this reaction are bases which have a pKa (at 25°C) of 8.5 to 20.0. Such bases include alkali metal hydroxides such as sodium or potassium hydroxide; alkali metal alkoxides such as sodium methoxide or potassium t-butoxide; alkali metal amides; amines such as diethylamine, hydroxylamine, ammonia and the like; and other common bases such as hydrazine and the like. At least one equivalent of base is needed for each protecting group.

The acyl protecting groups can also be removed with acid catalysts, such as methanesulfonic acid, hydrochloric acid, hydrobromic acid, sulfuric acid, or with acidic ion exchange resins. It is preferred to carry out such hydrolysis at relatively high temperature, such as the reflux temperature of the mixture, but temperatures as low as ambient may be used when particularly strong acids are used. Care must be taken to preserve these acyl protecting groups so that they are not prematurely cleaved during the early steps of the process of the invention.

The removal of ether protecting groups is carried out by known methods, for example, with ethanethiol and aluminum chloride.

The t-butyldimethylsilyl protecting group requires acid conditions, such as contact with gaseous hydrogen halide, for its removal.

Removal of the protecting groups may be conveniently carried out in alcoholic solvents, especially aqueous alkanols such as methanol. However, the deblocking reaction may also be carried out in any convenient solvent, such as polyols including ethylene glycol, ethers such as tetrahydrofuran, ketones such as acetone and methyl ethyl ketone, or dimethylsulfoxide.

In a preferred embodiment, the deblocking reaction employs ammonia to remove a benzoyl hydroxyprotecting group at a temperature of 10°C. It is preferable, however, to use an excess of base in this reaction, although the amount of excess base used is not crucial.

The resulting beta- enriched nucleoside 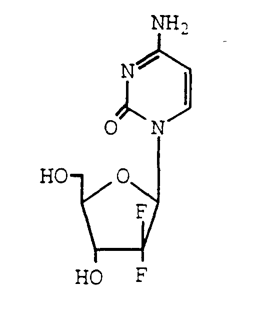 or an organic or inorganic acid addition salt thereof, may be extracted and/or isolated from the reaction mixture by the procedure described in U.S. Patent 4,965,374.

The following example illustrates specific aspects of the present invention and is not intended to limit the scope thereof in any respect and should not be so construed.

### Example 1

Preparation, purification and isolation of beta-anomer enriched 1-(2'-deoxy-2',2'-difluoro-3',5'-di-O-benzoyl-D-ribofuranosyl)-4-aminopyrimidin-2-one with 22.5 equivalents of bis-trimethylsilylcytosine

To a 250 ml 3-neck flask were added 30 g of cytosine, 25 mg of ammonium sulfate and 150 ml of hexamethyldisilazane, and the mixture was heated to 125°C and held for 30 minutes after the dissolution of all of the solids. Then the temperature was raised to 145°C, held until boiling stopped, and then held to 120°C under vacuum until solids began to form above the liquid level in the flask. Then the mixture was cooled to 105°C, and 25 ml of anisole was added.

In another 125 ml flask were combined 10 ml of anisole and 5.75 g of 2-deoxy-2,2-difluoro-3,5-dibenzoyl-D-ribofuranosyl-1-α-methanesulfonate, and the mixture was heated until a homogeneous liquid was formed. That liquid was added at constant temperature to the cytosine mixture, and the combined mixture was held at 100°C for 24 hours.

A 133 ml portion of 4N hydrochloric acid was placed in a 500 ml flask. A 31.3 ml portion of acetonitrile was added to the reaction mixture, and the diluted reaction mixture was then poured onto the acid with constant stirring, while a cooling bath was applied to the 500 ml flask. The combined mixture was then stirred at 70°C for 10 minutes, and was then filtered at constant temperature. The wet cake was then slurried for 10 minutes at 70°C with 25 ml of 4N hydrochloric acid, and was filtered again. That filter cake was slurried for 10 minutes at 70°C with 25 ml of deionized water, filtered, and the wet cake was slurried again at 70°C with 50 ml of deionized water. The pH of the aqueous slurry was raised to 7 with sodium bicarbonate, and the mixture was stirred for 10 minutes at 50°C or above, and was filtered again. That filter cake was slurried once more with 50 ml of deionized water at 70°C for 10 minutes, filtered, and the filter cake was dried and analyzed. It weighed 3.98 g, representing an isolated yield of 61 percent, and contained less than 1 percent of the undesired alpha-anomer.

## Claims

1. A process to purify and isolate a beta-anomer enriched nucleoside comprising:
a) providing a mixture containing R" and a beta-anomer enriched nucleoside of the formula 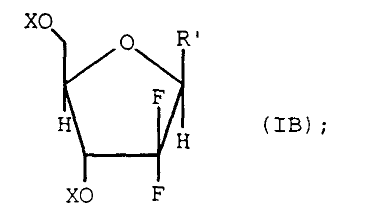 wherein each X is independently selected from hydroxy protecting groups and R' is a nucleobase of the formula 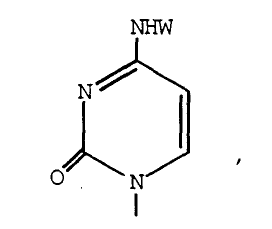 where W is an amino protecting group; and
R" is a nucleobase of the formula 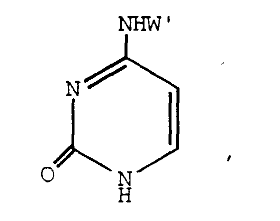 where W' is an amino protecting group or hydrogen; in a high boiling solvent;
b) diluting the mixture with acetonitrile;
c) adding the diluted reaction mixture to aqueous acid; and
d) holding the acid mixture so prepared at a temperature from 70°C to 100°C until the product of formula IB, where W is now W', has precipitated.

2. A process of claim 1 wherein the aqueous acid is hydrochloric acid from 1 N to 6 N.

3. A process of claim 1 wherein the acid mixture is agitated while the product is precipitating.

4. A process of claim 1 further including deblocking the purified formula IB product to form the nucleoside which is 1-(2'-deoxy-2',2'-difluoro-D-ribofuranosyl-4-aminopyrimidin-2-one.

## Patentansprüche

1. Verfahren zur Reinigung und Isolierung eines bezüglich Beta-Anomeren angereicherten Nucleosids, das die folgenden Stufen umfasst:
a) Bereitstellen eines Gemisches, das R" und ein bezüglich Beta-Anomeren angereichertes Nucleosid der folgenden Formel enthält; 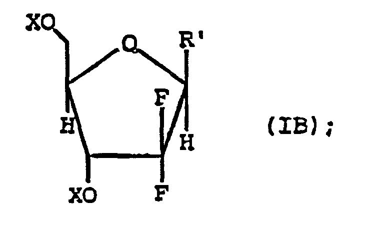 worin jeder Rest X unabhängig voneinander aus Hydroxyschutzgruppen ausgewählt ist und R¹ für eine Nucleobase der folgenden Formel steht: 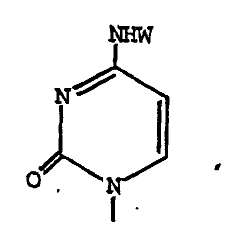 worin W für eine Aminoschutzgruppe steht und
R" für eine Nucleobase der folgenden Formel steht: 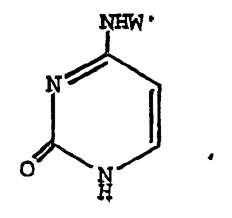 worin W' für eine Aminoschutzgruppe oder Wasserstoff steht,
in einem hochsiedenden Lösungsmittel;
b) Verdünnen des Gemisches mit Acetonitril.
c) Zugeben des verdünnten Reaktionsgemisches zu wässriger Säure und
d) Halten des so hergestellten sauren Gemisches bei einer Temperatur von 70 - 100 °C. bis das Produkt der Formel IB. worin W nun W' ist. ausgefallen ist.

2. Verfahren nach Anspruch 1, wobei die wässrige Säure Salzsäure mit einer Konzentration von 1 N bis 6 N ist.

3. Verfahren nach Anspruch 1, wobei das saure Gemisch gerührt wird, während das Produkt ausfällt.

4. Verfahren nach Anspruch 1, des weiteren umfassend ein Entblockieren des gereinigten Produkts der Formel IB unter Bildung des Nucleosids, bei dem es sich um 1-(2'-Desoxy-2',2'-difluor-D-ribofuranosyl-4-aminopyrimidin-2-on) handelt.

## Revendications

1. Procédé pour purifier et isoler un nucléoside enrichi en anomère bêta, comprenant :
a) la réalisation d'un mélange contenant un composé R" et un nucléoside enrichi en anomère bêta de formule 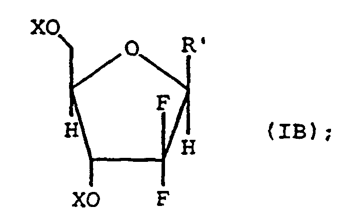 dans laquelle chaque groupe X est indépendamment choisi parmi les groupes de protection du groupe hydroxy et le groupe R' est une base nucléique de formule 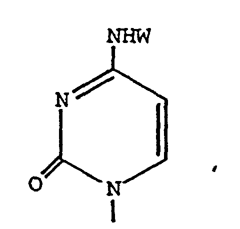 dans laquelle le groupe W est un groupe de protection du groupe amino et le composé R" est une base nucléique de formule 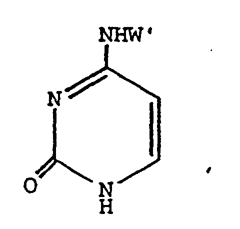 dans laquelle le groupe W' est un groupe de protection du groupe amino ou un atome d'hydrogène,
dans un solvant à point d'ébullition élevé ;
b) la dilution du mélange avec de l'acétonitrile ;
c) l'addition du mélange réactionnel dilué à un acide aqueux ; et
d) le maintien du mélange acide ainsi préparé à une température de 70°C à 100°C jusqu'à ce que le produit de formule IB, dans laquelle le groupe W est à présent égal au groupe W', ait précipité.

2. Procédé de la revendication 1, dans lequel l'acide aqueux est de l'acide chlorhydrique de 1 N à 6 N.

3. Procédé de la revendication 1, dans lequel le mélange acide est agité pendant la précipitation du produit.

4. Procédé de la revendication 1, comprenant en outre le déblocage du produit purifié de formule IB pour former le nucléoside qui est de la 1-(2'-désoxy-2',2'-difluoro-D-ribofurannosyl)-4-aminopyrimidin-2-one.
